# EUROPEAN PATENT SPECIFICATION

(11) **EP 2 959 232 B1**
(45) Date of publication and mention of the grant of the patent: **10.10.2018**
(21) Application number: 14712352.5
(22) Date of filing: 24.02.2014
(51) Int. Cl.: F24F 3/16, A61L 9/20, B01D 53/00

(54) **DEVICE FOR AIR FILTRATION AND PURIFICATION**
VORRICHTUNG ZUR LUFTFILTERUNG UND -REINIGUNG
DISPOSITIF POUR FILTRATION ET PURIFICATION D'AIR

(30) Priority: 24.02.2013 PT 10680613
(43) Date of publication of application: 30.12.2015
(73) Proprietor: Vieira & Lopes Lda, 4700-023 Braga (PT)
(72) Inventor: SOARES PINHEIRO LOPES, Marco André, P-4990-511 Ponte De Lima (PT); FERREIRA DE SOUSA , Rafael Simão, P-4810-446 Guimaraes (PT)
(74) Representative: Silvestre de Almeida Ferreira, Luís Humberto
(86) International application number: PCT/IB2014/059219
(87) International publication number: WO 2014/128673

(56) References cited:
- WO-A1-96/37281
- JP-A- H10 244 829
- JP-A- 2010 279 462
- US-A1- 2004 251 122
- US-A1- 2009 010 801

## Description

### Technical field

The present disclosure relates to a device and method of filtration and purification of air. More particularly, this invention relates to an air purifier device for air handling units (AHUs) and/or ventilation ducts for, but not limited to, clinical, industrial and/or commercial use.

### Background Art

Nowadays, people pass 90% of their time in closed spaces and, therefore, an increasing care in the creation and upkeep of healthier environments as a personal and as professional level become a major issue. Some attempts have being made to achieve the purification of air such as the ones described in the following patents:

US 7704463 B2 - A UV light system for use in a central air handling unit of a heating or air conditioning system includes a UV light source and is adapted for operation on, and receives power from, an approximately 24 VAC low voltage power supply for a thermostat of heating or air conditioning system.

US 005933702A - A method for disinfecting an air stream containing microorganisms including the steps of providing an air stream containing microorganisms having a relative humidity greater than about 40 %; and contacting the air stream with a photo-catalyst having predetermined band gap energy in the presence of a source of photons having wavelength corresponding to the band gap energy of the photo-catalyst, so that at least a portion of the microorganisms in the air stream are destroyed by photo-catalytic oxidation.

US 6248235; US 6261449; US 6274049 and US 6524457 - A photo-catalytic oxidation purification system includes an ultraviolet light source and a filter that comprises a pleated wire mesh substrate with nanophase metal oxide oxidation catalyst suspended on the substrate, wherein the catalyst is applied without an adhesive using an electromechanical plating process. As a fluid containing organic contaminants is directed though the filter in the presence of ultra violet light from the light source, the catalyst oxidizes and decomposes the organic contaminants into environmentally harmless components.

US 2012/0283508 A1 - Purified air is provided, having TVOC content of from less than 5 ppb to about 500 ppb, a biological content of from less than 1 CFU/m³ to 150 CFU/m³ and a particulate content of from about 1000 0,3 µm particles per ft³ to about 5000 0,3 µm particles per ft³, or from about 600 0,5 µm particles per ft³ to about 500000 0,5 µm particles per ft³.

US 2010/0254868 A1 - A system and method for purifying a fluid (such as air or water) containing contaminants includes removing the contaminants from the fluid using a capturing device, such as an adsorbent and/or a particle filter. The contaminants may include volatile organic compounds (VOCs) and microorganisms. The method further includes generating ozone molecules using an ozone generating device.

US 2010/0172793 A1 - An air purification system for a heating, ventilation, and air conditioning (HVAC) system includes an ozone generating device that is used to introduce ozone into an air stream flowing through the ozone generating device. The ozone is used to remove contaminants, including volatile organic compounds (VOCs), from the air stream.

US 2002/0062739 A1 - An electronic air cleaner assembly for attachment to an air handling unit of an air-conditioning system has a housing containing a plurality of electrostatic filter cells. One side wall of the lousing is pivotally mounted to a side of the AHU by hinges and the opposite side wall is releasably attached to the opposite wall of the AHU by a releasable locking device

US 2009/010801 discloses a device for air filtration and purification upon which the preamble of appending claim 1 is based.

Having as basis the available and known purification systems and methods, the hereby air purification system achieves to overcome prior art shortcomings in the treatment and purifications of indoor air in HVAC, air conditioning and heating systems.

### Disclosure of the Invention

Until know the possible similar equipment using two for industrial equipments, and in some cases 3, of the filtration and purification phenomena only were used for commercial/small air volumes (maximum 2.000 m3 of air) - home applications. This present air purification system solves the upscale problem, regarding the inner geometry of the components in use the three phenomena together in just one equipment, minimize to usable pressure drop created by any filtration/purification system at the work scale of AHUs industry, health care facilities and being an add to high efficient commercial use. This purification system also solves the energy efficiency problem of all industrial scale equipment, as it is engineered to use UVC/VUV LEDs that consume less than 80% energy then similar low pressure mercury vapor lamps, known as UVGI (ultraviolet germicidal irradiation) lamps. The air purification system also reformulates the electrostatic precipitator shape to be optimized to the high air flow, UVC/VUV disinfection and photo-catalytic ionization phenomena.

The existent systems have a passive and obstructive geometry for air purification. By placing components perpendicularly to the airflow direction, they create higher pressure drops with higher energy consumptions to overcome those barriers. Also the available system use UVGI lamps, that use low pressure mercury vapor that is carcinogenic and does not emit UVC radiation (it emits at 254 nm) in the perfect wavelength to disinfect microbial load, that in this case in 265 nm. Another point are the geometries of the photo-catalytic surfaces that are not engineered to allow the maximum absorbency to its surfaces and by consequence do not purify perfectly the air from VOCs. Furthermore, the ones that maximize contact, placing the photo-catalytic surfaces perpendicularly to the airflow direction, create higher pressure drops in the system, increasing dramatically the energy consumption to disinfect the same amount of pollutant.

Based on these system and efficiency gaps, the present geometry and assembly of the air purification system seeks to optimize the three phenomena to:
- Maximize disinfection by UVC/VUV using minimum energy possible, mainly to control, inhibit and destroy all microbial (example: bacteria, fungus and virus) replication and activity;
- Maximize VOCs disinfection using photo-catalytic ionization of air to oxidize the air pollutants, maximizing the superficial area of semiconductor coat and maximum contact of VOCs with all the photo-catalytic surfaces with attention to maximum exposure of the semiconductor surfaces to the UVC/VUC radiation that initiates the photo-catalytic ionization phenomenon;
- Maximize the residential time of the microbial and pollutants particle inside the air purifications system by adding electrostatic precipitation phenomenon, redesigned to meet the disclosed geometry and still maximizing particle capture in the wave shape inner plates also coated with a photo-catalytic semiconductor layer;
- Minimum pressure drop during airflow as consequence of the disclosed geometry that aims to maximize purification phenomena while allowing the airflow to pass through it with minimum energy required (wave shape and perforated inner plates);
- Does not produce any contaminants such as ozone (O₃) by the system operation (controlled UV wavelength to ensure no production of other contaminants).

The present air purification system seeks to treat more volume of air, with less pressure drop and energy consumptions, for the major air pollutants: bacteria, fungus, virus, VOCs and airborne particle.

The present invention is an industrial scale air purifier than may be applied to, but not limited to, in AHUs for clinical, industrial and/or commercial use, also passive of installation in ventilation ducts. The system can be used in recirculation flow and/or one passage flow, for microbiological, volatile organic compounds (VOCs) and airborne particle filtration, decontamination and/or inactivation. Three phenomena are utilized in the system, namely: UVC (ultraviolet electromagnetic radiation subtype C) and VUV (vacuum ultraviolet radiation that is absorbed by air), photo-catalytic ionization of air through a semi-conductive layer coated in the inner surfaces of the system and by electrostatic precipitation of particles. The three phenomena were assembled together to maximize the purification process and system's geometry was engineered to produce the minimum pressure drop during air passage and still produce maximum available air purification. All the inner components of the air purification are preferably in aluminium and/or stainless steel to maximize UVC and VUV reflection and shield effect.

It is disclosed a device for filtration and air purification comprising:
- the use of three purification phenomena simultaneously, UVC (Ultraviolet electromagnetic radiation subtype C) and/or VUC (Vacuum ultraviolet radiation that is absorbed by air) and photo-catalytic ionization of air used for industrial applications; and
- the inner part being a cubic shape box with 4 inner wave shape plates coated with semi-conductive materials coupled each to its electrostatic precipitator system.

It is disclosed a device for air filtration and purification comprising:
an enclosure for the flow of the air, said enclosure comprising an air inlet opening and an air outlet opening;
one or more inner plates arranged inside said enclosure at an inclined angle in respect of the air flow direction, between said air inlet and outlet, and arranged such that the air flows along such inclined inner plate or plates;
one or more UV light emitters arranged inside said enclosure to emit UV light over said inner plates and over said air flow;
wherein said inner plates are photo-catalytic and are electrostatic precipitators.

According to the invention, the direction of the inclined angle of the inner plate, in respect of the air flow direction, is the inclined angle of the inner plate, in respect of a horizontal plane.

The invention furthermore comprises one or more pairs of said inner plates, wherein each pair of inner plates is placed in a cross-shape arrangement with the two plates being arranged inclined and side-by-side transversally to the airflow.

In an embodiment, the pairs of inner plates are placed linearly along the direction of the airflow, wherein the pairs of inner plates overlap with the neighbouring pair of inner plates in the direction of the airflow.

In an embodiment, the inclined angles of the two plates, of each pair of plates, are supplementary angles in respect of the air flow direction, i.e. angles that total 180°.

In an embodiment, the two plates, of each pair of inner plates, are positioned at 45º from the bottom surface of the enclosure and at a right angle to the other plate of the pair.

In an embodiment, said enclosure is photo-catalytic.

In an embodiment, said inner plates or enclosure are coated with a VOC photo-catalytic layer.

In an embodiment, said layer is semi-conductive.

In an embodiment, said layer comprises titanium oxide.

In an embodiment, one or more of the inner plates are perforated such that pressure drop along the air flow is decreased, or a portion of UV light permeates the inner plate, or the pressure drop along the air flow is decreased and a portion of UV light permeates the inner plate.

In an embodiment, one or more of the inner plates has a wave shape such that the superficial area for air particle contact is increased.

In an embodiment, the wave shape is a wave shaped profile transversal to the airflow or the wave shape is a wave shaped profile longitudinal to the airflow.

An embodiment further comprises ionizer filaments for creating electrostatic precipitation in each inner plate, wherein said filaments for each inner plate are arranged on a plane parallel to said plate and located between the air inlet and said plate.

In an embodiment, said ionizer filaments are located on top of the side towards the airflow of each said plate.

In an embodiment, the ionizer filaments comprises tungsten or stainless steel.

In an embodiment, the UV light emitters are arranged at the top and bottom of the enclosure.

In an embodiment, the UV light emitters are UVC and VUV light emitters.

In an embodiment, the UV light emitters are UVC/VUV LEDs.

In an embodiment, the inner components of the device comprise UVC and VUV reflectance materials, in particular aluminium or stainless steel.

It is also disclosed an air handling unit AHU or ventilation duct comprising any of the above disclosed devices for filtration and air purification.

In an embodiment not forming part of the claims, said air handling unit AHU or ventilation duct comprises a removable panel and rails for sliding in or out said device for filtration and air purification.

In an embodiment, the cross shape between each two inner plates are positioned an oblique position (45º from the bottom surface) and make a right angle between each two plates.

In an embodiment, the plates present a wave shape to increase the superficial area which will contact with all air particles.

In an embodiment, all the inner components of the air purification are in UVC/VUV reflectance materials namely aluminium and/or stainless steel to maximize UVC and/or VUV reflection and shield effect.

In an embodiment, the ionizer filaments are made with tungsten, stainless steel or other material as polarization filament in electrostatic precipitation phenomenon.

In an embodiment, the plates are perforated to decrease pressure drop during airflow and allow intercommunication of UVC/VUC photons emitted by each source. The perforations also enable the UV light to further permeate the device avoiding the existence of dark areas in terms of UV light.

In an embodiment, the ionizer filaments are placed on the top of each inner plate to create electrostatic precipitation.

In an embodiment, the outer box has a removable panel to slide in the air purification system.

### Brief Description of Drawings

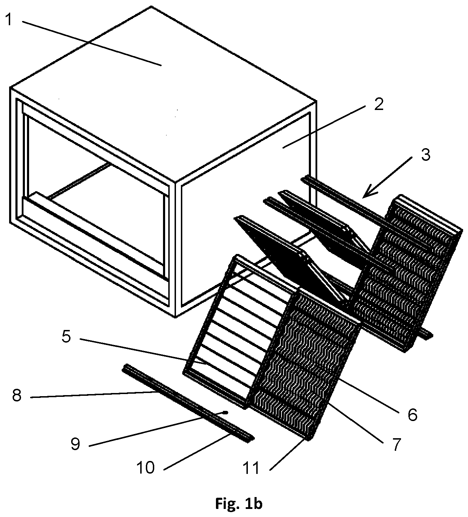

The following figures provide preferred embodiments for illustrating the description and should not be seen as limiting the scope of invention.

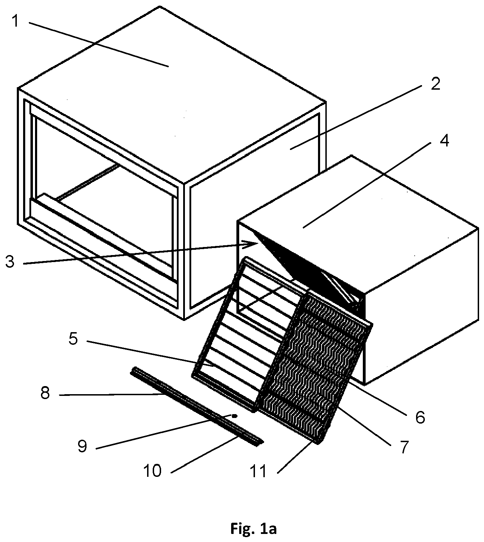

**Figure 1a****-b** describes in exploded view all components of an embodiment of the air purification system, namely the UVC/VUV source (in this configuration using LEDs (Light-Emitting Diodes), the photo-catalytic surfaces coated with a semiconductor (all inner surfaces are coated with the semiconductor) and the electrostatic precipitation elements (the ionizer filaments and the collector plates with wave shape).
   The exploded view of the air purification system in figure 1 shows: 1 - modular AHUs box (shell of the air purification system); 2 - side door of AHU box for insertion of the air purification system; 3 - cross geometry of the inner plates (each plate occupies half of the cut) to minimize pressure drop and still maximize all the purification phenomena; 4 - inner air purification systems' box, coated with photo-catalytic surface with semiconductor; 5 - ionizer filaments of the electrostatic precipitator element; 6 - perforation fill of the inner plates, to minimize pressure drop and allow intercommunication of UVC photons; 7 - wave shape inner electrostatic collect plate to maximize superficial area and coated with photo-catalytic semiconductor; 8 - linear pattern of UVC/VUV LEDs; 9 - UVC/VUV LEDs source; 10 - support gutter for LEDs; 11 - support gutters for inner plates and electrostatic precipitation elements.

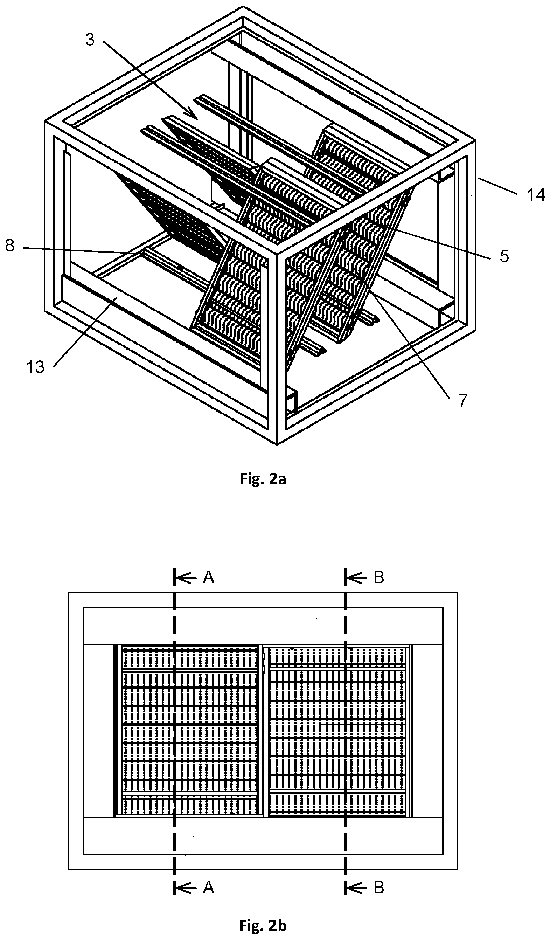

**Figure 2a****-d** exposes isometric, face and section cut views of an embodiment of the air purification system, allowing the visualization of the cross geometry between the inner plates, the location of the LEDs strips and electrostatic precipitation elements.
   The views of the air purification system show: 3 - cross geometry between inner plates (each plate occupies half of the section cut) to minimize pressure drop and still maximize all the purification phenomena; 5 - ionizer filaments location (placed in front of each wave shape collector plate); 7 - wave shape inner electrostatic collect plate to maximize superficial area and coated with photo-catalytic semiconductor; 8 - linear pattern of UVC/VUV LEDs strips (for prototype for 5000 m3/h, 16 LEDs); 13 - air inlet section; 14 - air outlet section.

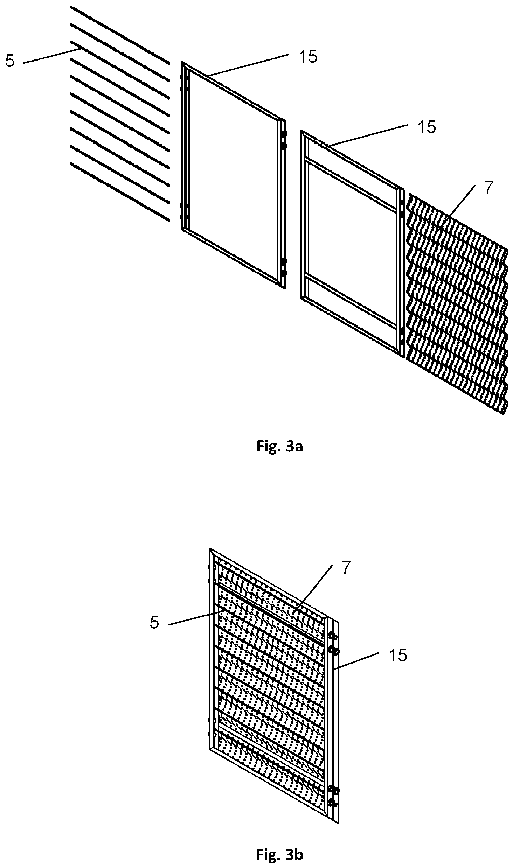

**Figure 3a****-b** shows a schematic representation of an embodiment of the inner plates, either dismounted (3a) or mounted (3b), comprising: 5 - ionizer filaments location (placed in front of each wave shape collector plate); 7 - wave shape inner electrostatic collect plate to maximize superficial area and coated with photo-catalytic semiconductor; 15 - mounting frames.

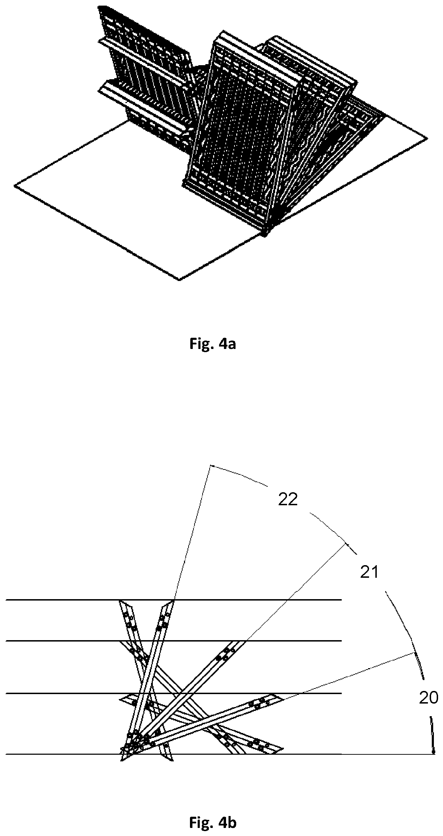

**Figure 4a****-b** shows a schematic representation of an embodiment of varying mounting angles of the inner plates, with angles from the airflow line varying from 20° (20) to 70°, including approximately 45° (21).

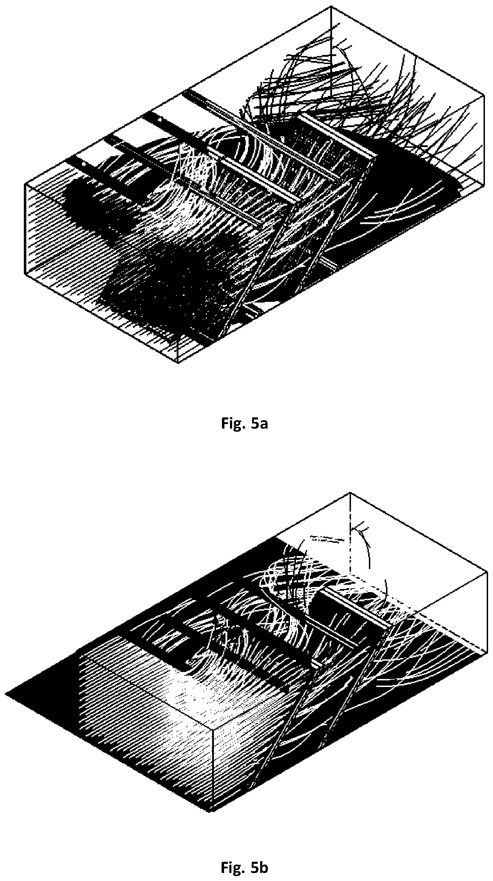

**Figure 5a****-b** shows a schematic representation of simulation tests of an embodiment both in terms of pressure (5a) and velocity (5b), displaying the 'S'-shaped airflows of the embodiment.

### Detailed description of the Invention

The system was engineered to purify high volumes of air in recirculation and/or one passage flow. Using three purification phenomena, namely: UVC and VUV radiation, photo-catalytic ionization of air by semiconductor layer and electrostatic precipitation, the system was engineered in its geometry and phenomena power to maximize air purification from microbial load (bacteria, fungus, spores and virus), VOCs and particles with minimum pressure drop. The system was developed to be used as a component of an AHU, however it can be applied to existent AHUs and ventilation ducts. The outer box (AHU module) has a removable panel to slide in the air purification system for an easier installation and maintenance. For this, a rail system is present inside the AHU box that tightly holds the inner air purification system.

Focusing in the inner part of the system: it is composed by a cubic shape box with 4 inner wave shape plate coupled each to its electrostatic precipitator system, and by UVC/VUC LEDs (quantity size dependent, although for the prototype modelled for a flow of 5000 m³/h, are present 16 LEDs). All the inner metallic components are made of high UVC reflectance materials, namely aluminium or stainless steel, with exception to the ionizer filaments that can also be made with tungsten.

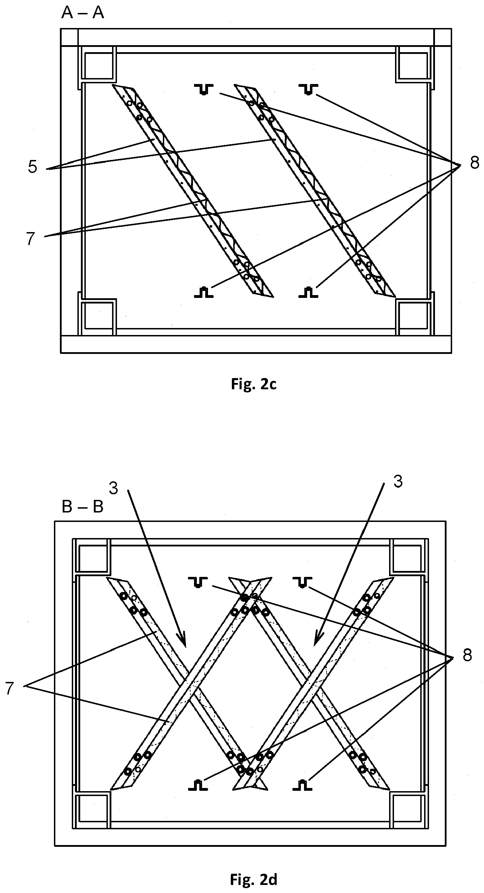

Geometrically the system was design to let the air pass with minimum pressure drop and maximum purification process. To achieve that, the proposed geometry is a cross shape arrangement between each two inner plates that fill a section cut of the air passage section. The inner plates are positioned an oblique position (± 45 º from the bottom surface) and make a right angle between each two plates (3). To maximize residence time and purification phenomena a second row of plates are placed after the first two, with the same configuration, making the total 4 inner plates (Figure 2 - section cut A-A and B-B). The wave shape of the inner plates was engineered to increase the superficial area which will contact with all air particles (microbial and VOCs included) (7). This geometry increases around 20% the superficial area compared to a linear sheet of metal. To this plate, perforation around 30% is made in order to decrease pressure drop during airflow and allow intercommunication of UVC/VUV photons emitted by each source (6, 7).

On top of each inner plate are placed the ionizer filaments (5) to create electrostatic precipitation phenomenon in each plate and design to the wave shape collector plate.

To produce and initiate the UVC/VUC and photo-catalytic ionization of air, strips of UVC/VUV LEDs are placed in the bottom and top inner surfaces, fixed to gutters to firmly secure the LEDs (8, 9). This LEDs configuration allows maximum UV exposure of all inner surfaces to create the photo-catalytic ionization of air and ensure that all particles travelling inside the air purification system suffer sufficient photochemical reactions with UV photons during maximum time possible.

Introducing the electrostatic precipitation phenomenon in each inner wave plate is possible to trap and treat more efficiently microbial particles and increase the residential time inside the purification system, increasing its performance (5). This phenomenon also adds the advantage of filter particle of larger diameter (like dust).

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure is of course not in any way restricted to the embodiments described and a person with ordinary skill in the art will foresee many possibilities to modifications thereof without departing from the basic idea of the disclosure as defined in the appended claims.

The above described embodiments are obviously combinable.

The following dependent claims set out particular embodiments of the disclosure.

## Claims

1. A device for air filtration and purification comprising:
an enclosure for the flow of the air, said enclosure comprising an air inlet opening and an air outlet opening;
one or more inner plates (7) arranged inside said enclosure at an inclined angle in respect of the air flow direction, between said air inlet and outlet, and arranged such that the air flows along such inclined inner plate or plates;
one or more UV light emitters (8) arranged inside said enclosure to emit UV light over said inner plates and over said air flow;
wherein said inner plates are photo-catalytic and are electrostatic precipitators;
**characterized in that**
the direction of the inclined angle of the inner plate, in respect of the air flow direction, is the inclined angle of the inner plate, in respect of a horizontal plane;
the device comprising one or more pairs of said inner plates, wherein each pair of inner plates is placed in a cross-shape arrangement with the two plates being arranged inclined and side-by-side transversally to the airflow.

2. Device according to the previous claim wherein the pairs of inner plates are placed linearly along the direction of the airflow, wherein the pairs of inner plates overlap with the neighbouring pair of inner plates in the direction of the airflow.

3. Device according to any of the claims 1-2 wherein the inclined angles of the two plates, of each pair of plates, are supplementary angles in respect of the air flow direction, i.e. angles that total 180°.

4. Device according to any of the claims 1-3 wherein the two plates, of each pair of inner plates, are positioned at 45° from the bottom surface of the enclosure and at a right angle to the other plate of the pair.

5. Device according to any of the previous claims wherein said enclosure is photo-catalytic.

6. Device according to any of the previous claims wherein said inner plates are coated with a VOC photo-catalytic layer, in particular said layer is semi-conductive, further in particular said layer comprises titanium oxide.

7. Device according to any of the previous claims wherein one or more of the inner plates are perforated such that pressure drop along the air flow is decreased, or a portion of UV light permeates the inner plate, or the pressure drop along the air flow is decreased and a portion of UV light permeates the inner plate.

8. Device according to any of the previous claims wherein one or more of the inner plates has a wave shape such that the superficial area for air particle contact is increased.

9. Device according to the previous claim wherein the wave shape is a wave shaped profile transversal to the airflow or the wave shape is a wave shaped profile longitudinal to the airflow.

10. Device according to any of the previous claims further comprising ionizer filaments for creating electrostatic precipitation in each inner plate, wherein said filaments for each inner plate are arranged on a plane parallel to said plate and located between the air inlet and said plate.

11. Device according to any of the previous claims wherein said ionizer filaments are located on top of the side towards the airflow of each said plate, further in particular the ionizer filaments comprise tungsten or stainless steel.

12. Device according to any of the previous claims wherein the UV light emitters are arranged at the top and bottom of the enclosure, in particular the UV light emitters are UVC and VUV light emitters, further in particular the UV light emitters are UVC/VUV LEDs.

13. Device according to any of the previous claims wherein the inner components of the device comprise UVC and VUV reflectance materials, in particular aluminium or stainless steel.

14. Air handling unit AHU or ventilation duct comprising the device for filtration and air purification according to any previous claim.

## Patentansprüche

1. Eine Vorrichtung zur Luftfilterung und -reinigung, umfassend:
- ein Gehäuse für den Luftstrom, wobei das genannte Gehäuse eine Lufteintrittsöffnung und eine Luftaustrittsöffnung aufweist;
- eine oder mehrere innere Platten (7), die innerhalb des genannten Gehäuses in einem geneigten Winkel in Bezug auf die Strömungsrichtung der Luft zwischen dem genannten Lufteinlass und Luftauslass angeordnet sind und zwar so, dass die Luft entlang einer oder mehrerer dieser geneigten inneren Platten strömt;
- eine oder mehrere UV-Strahlungsquellen (8), die innerhalb des genannten Gehäuses angeordnet ist/sind, um die genannten inneren Platten und den genannten Luftstrom mit UV-Licht zu bestrahlen;
wobei die genannten inneren Platten photokatalytisch und elektrostatische Abscheider sind; **dadurch gekennzeichnet, dass**
die Ausrichtung des geneigten Winkels der inneren Platte in Bezug auf die Strömungsrichtung der Luft dem Neigungswinkel der inneren Platte in Bezug auf eine horizontale Ebene entspricht;
die Vorrichtung ein oder mehrere Paare der genannten inneren Platten umfasst, wobei jedes Paar der inneren Platten kreuzförmig angeordnet ist, wobei die beiden Platten geneigt und nebeneinander quer zum Luftstrom angeordnet sind.

2. Vorrichtung nach dem vorangehenden Anspruch, bei der die inneren Plattenpaare linear entlang der Strömungsrichtung der Luft angeordnet sind, wobei sich die inneren Plattenpaare mit dem benachbarten inneren Plattenpaar in Strömungsrichtung der Luft überlappen.

3. Vorrichtung nach einem der Ansprüche 1-2, wobei die geneigten Winkel der beiden Platten jedes Plattenpaars zusätzliche Winkel in Bezug auf die Strömungsrichtung der Luft sind, d. h. Winkel mit insgesamt 180°.

4. Vorrichtung nach einem der Ansprüche 1-3, wobei die beiden Platten jedes Paares der inneren Platten mit 45° zur Unterseite des Gehäuses und im rechten Winkel zur anderen Platte des Paares angeordnet sind.

5. Vorrichtung nach einem der vorangehenden Ansprüche, wobei das Gehäuse photokatalytisch ist.

6. Vorrichtung nach einem der vorangehenden Ansprüche, wobei die genannten inneren Platten mit einer photokatalytischen VOC-Schicht überzogen sind, die Schicht insbesondere halbleitend ist und die Schicht ferner insbesondere Titanoxid enthält.

7. Vorrichtung nach einem der vorangehenden Ansprüche, wobei eine oder mehrere der inneren Platten perforiert sind, sodass der Druckabfall entlang des Luftstroms verringert wird oder ein Teil des UV-Lichts die innere Platte durchdringt oder der Druckabfall entlang des Luftstroms verringert wird und ein Teil des UV-Lichts die innere Platte durchdringt.

8. Vorrichtung nach einem der vorangehenden Ansprüche, wobei eine oder mehrere der inneren Platten eine Wellenform aufweisen, sodass die Oberfläche für den Kontakt mit den Luftpartikeln vergrößert wird.

9. Vorrichtung nach dem vorangehenden Anspruch, wobei die Wellenform ein wellenförmiges Profil quer zum Luftstrom ist oder die Wellenform ein wellenförmiges Profil längs zum Luftstrom ist.

10. Vorrichtung nach einem der vorangehenden Ansprüche, die ferner lonisationsdrähte umfassen, um eine elektrostatische Abscheidung an jeder inneren Platte hervorzurufen, wobei die genannten Drähte bei jeder innere Platte auf einer Ebene parallel zu der genannten Platte sowie zwischen dem Lufteinlass und der genannten Platte angeordnet sind.

11. Vorrichtung nach einem der vorangehenden Ansprüche, wobei die lonisationsdrähte auf der dem Luftstrom zugewandten Oberseite jeder Platte angeordnet sind und die lonisationsdrähte ferner insbesondere Wolfram oder Edelstahl umfassen.

12. Vorrichtung nach einem der vorangehenden Ansprüche, wobei die UV-Strahlungsquellen oben und unten im Gehäuse angeordnet sind, wobei die UV-Strahlungsquellen insbesondere UVC- und Vakuum-UV-Strahlungsquellen sind, wobei die UV-Strahlungsquellen ferner insbesondere UVC/Vakuum-UV-LEDs sind.

13. Vorrichtung nach einem der vorangehenden Ansprüche, wobei die inneren Komponenten der Vorrichtung UVC- und VUV reflektierende Materialien umfassen, insbesondere Aluminium oder Edelstahl.

14. Lüftungsgerät (AHU) oder Lüftungskanal umfassend die Vorrichtung zur Filterung und Luftreinigung nach einem der vorangehenden Ansprüche.

## Revendications

1. Un dispositif pour la filtration et purification d'air comprenant:
- un boîtier pour la circulation de l'air, ledit boîtier comprenant une ouverture d'entrée d'air et une ouverture de sortie d'air ;
- une ou plusieurs plaques internes (7) disposées à l'intérieur dudit boîtier à un angle incliné en respect la direction du débit d'air, entre ladite entrée et sortie d'air, et disposées de façon à ce que l'air circule le long de ladite plaque ou desdites plaques inclinées ;
- un ou plusieurs émetteurs de lumière UV (8) disposé(s) à l'intérieur dudit boîtier pour émettre une lumière UV sur lesdites plaques internes et sur ledit débit d'air ;
dans lequel lesdites plaques internes sont photocatalytiques et sont des précipitateurs électrostatiques ;
charactérisé en ce que
la direction de l'angle incliné de la plaque interne, en respect de la direction du débit d'air, est l'angle incliné de la plaque interne, en respect d'un plan horizontal ;
le dispositif comprenant une ou plusieurs paires desdites plaques internes, dans lequel chaque paire de plaques internes est placée en un arrangement en forme de croix, les deux plaques étant disposées de façon inclinée et côte-à-côte transversalement au débit d'air.

2. Dispositif selon la revendication précédente, dans lequel les paires de plaques internes sont placées linéairement le long de la direction du débit d'air, dans lequel les paires de plaques internes se superposent avec la paire voisine de plaques internes dans la direction du débit d'air.

3. Dispositif selon l'une quelconque des revendications 1-2, dans lequel les angles inclinés des deux plaques, de chaque paire de plaques, sont des angles complémentaires en respect de la direction du débit d'air, c'est-à-dire, angles qui représentent un total de 180º.

4. Dispositif selon l'une quelconque des revendication 1-3, dans lequel les deux plaques, de chaque paire de plaques internes, sont positionnées à 45º par rapport à la surface inférieure du boîtier et à un angle droit par rapport à l'autre plaque de la paire.

5. Dispositif selon l'une quelconque des revendications précédentes, dans lequel ledit boîtier est photocatalytique.

6. Dispositif selon l'une quelconque des revendications précédentes, dans lequel lesdites plaques internes sont recouvertes d'une couche photocalatytique de COV, ladite couche étant en particulier semi-conductrice, ladite couche comprenant plus particulièrement de l'oxyde de titane.

7. Dispositif selon l'une quelconque des revendications précédentes, dans lequel une ou plusieurs des plaques internes sont perforées de façon à ce que la chute de pression le long du débit d'air soit réduite, ou qu'une partie de la lumière UV pénètre la plaque interne, ou que la chute de pression le long du débit d'air soit réduite et qu'une partie de la lumière UV pénètre la plaque interne.

8. Dispositif selon l'une quelconque des revendications précédentes, dans lequel une ou plusieurs des plaques internes ont une forme ondulée telle que la surface superficielle pour le contact de particules d'air soit accru.

9. Dispositif selon la revendication précédente, dans lequel la forme ondulée est un profil de forme ondulée transversal au débit d'air ou la forme ondulée est un profil de forme ondulée longitudinal au débit d'air.

10. Dispositif selon l'une quelconque des revendications précédentes comprenant également des filaments ioniseurs pour créer une précipitation électrostatique dans chacune des plaques internes, dans lequel lesdits filaments pour chacune des plaques internes sont disposés en un plan parallèle à ladite plaque et situés entre l'entrée d'air et ladite plaque.

11. Dispositif selon l'une quelconque des revendications précédentes, dans lequel lesdits filaments ioniseurs se situent sur le haut de la latérale en direction du débit d'air de chacune desdites plaques, les filaments ioniseurs comprenant plus particulièrement du tungstène ou de l'acier inoxydable.

12. Dispositif selon l'une quelconque des revendications précédentes, dans lequel les émetteurs de lumière UV sont disposés sur le haut et le bas du boîtier, les émetteurs de lumière UV étant en particulier des émetteurs de lumière UVC et VUV, les émetteurs de lumière UV étant plus particulièrement des LEDs UVC/VUV.

13. Dispositif selon l'une quelconque des revendications précédentes, dans lequel les composants internes du dispositif comprennent des matériaux de reflectance UVC et VUV, en particulier de l'alluminium et de l'acier inoxydable.

14. Unité de traitement d'air AHU ou conduit de ventilation comprenant le dispositif pour la filtration et purification d'air d'après l'une quelconque des revendications précédentes.
